# EUROPEAN PATENT APPLICATION

(11) **EP 3 858 998 A1**
(43) Date of publication of application: **04.08.2021**
(21) Application number: 19856461.9
(22) Date of filing: 13.03.2019
(51) Int. Cl.: C12N 15/90, C12N 5/10

(54) **MRFFT1 CELL**

(30) Priority: 30.09.2018 CN 201811153232
(71) Applicant: Beijing DCTY Biotech Co., Ltd., Beijing 102200 (CN)
(72) Inventor: JIAO, Shuchang, Beijing 100000 (CN); ZHANG, Rong, Beijing 100000 (CN); ZHOU, Zishan, Beijing 100000 (CN); SONG, Yuji, Beijing 100000 (CN); XIE, Jiasen, Beijing 100000 (CN); ZHANG, Tianfu, Beijing 100000 (CN); WANG, Haiyan, Beijing 100000 (CN); YUAN, Han, Beijing 100000 (CN)
(74) Representative: Lang, Christian
(86) International application number: PCT/CN2019/077924
(87) International publication number: WO 2020/062794

(57) **Abstract**

The Invention relates to a MRFFT1 cell and a preparation method thereof. The Invention screens out mutant polypeptides with prediction of antigenic determinants, and links and synthesizes the expression gene sequences of mutant polypeptides. At the same time, the MVA virus vector is constructed to package MVA virus, and APC cells are transfected to complete the modification of specific MV cells, and PBMCs isolated from peripheral blood are co-cultured in vitro to screen out the effective polypeptide, and ordinary T cells is transformed into RFF cells with more precise killing ability through the second pulse of precise effective polypeptide stimulation. Then, the principle of TCR-T technology is used to modify the T cells, and gene coding technology is used to knock out the immunosuppressive target of the modified T cells. The specific killing T cells are precisely protected from in vivo inhibition, and the cytotoxicity of T cells to tumor cells is improved.

## Description

### TECHNICAL FIELD:

The invention discussed in this paper (hereinafter "Invention") falls under the biotech realm, and it is concerned with a specific species of MRFFT1 cell and the related preparation procedures.

### BACKGROUND ART:

It has been proven that existing LAK, DC, CIK, DC-CIK cells and associated methods largely ineffective in specific cancer immunotherapy at present, while the technics relating to NK, CAR-NK, TIL cells have yet to mature. In addition, there are still many shortcomings in the safety and efficiency of CAR-T therapy for treatment of solid tumor in clinical trials.

The existing technology generally produces a specific killing by transforming DC cells to T cells. By using viruses as vectors to transfect presenting T cells, some laboratories could induce the specific killing of T cells. In our previous study, we have also directly stimulated PBMC with mutant mixed polypeptide to induce T cells. Other laboratories use TCR-T technology to deliver MAGE A3 antigen.

The above therapeutic methods are not mature, especially in the theoretical study of inducing DC cells and DC cells to load tumor antigens in vitro. However, there are still many problems in the process of specific implementation, such as the lack of clear molecules related to the occurrence and development of tumor cells as induced antigens. Because the tumor antigens are unknown and the immunosuppression of tumor microenvironment is impaired, it is difficult to implement specific immunotherapy based on cells. In addition, although some methods involve T cell priming by antigens in vitro, they are difficult to induce effective T cell killing without co-stimulation and amplification, because these specific cells can't infiltrate into the tumor microenvironment successfully and remain cytotoxicity for a long time. Others can also be presented and co-cultured in vitro, but the target is single (MAGE-3), only effective on individual cancers such as non-small cell lung cancer. Although there are some methods of transfection and presentation using lentivirus as vector, the safety and convenience are not as good as that of polypeptide method. The direct stimulation of simple mixed polypeptides is simple and convenient, but its efficiency is low. Secondary stimulation of specific precision polypeptide is not as direct as that of tumor specific antigen transduced by T cell receptor. The existing TCR-T lacks accurate TCR to cover more tumor species in solutions for the treatment of hematological and solid tumors.

These methods do not take into account of the self-defense technology of T cells so that a limited amount of specific T cells is exposed directly to a complex and severe tumor immunosuppressive microenvironment.

### SUMMARY OF THE INVENTION:

The Invention uses human peripheral blood for ctDNA sequencing or tumor tissue for the whole exome sequencing, screen mutation sites for antigen epitope prediction, and link to the synthesized mutant polypeptides for express the gene line. At the same time, the MVA virus vector is constructed to package MVA virus, and APC cells are transfected to complete the modification of specific MV cells, and PBMCs isolated from peripheral blood are co-cultured in vitro to screen out the effective polypeptide, and ordinary T cells are transformed into RFF cells with more precise killing ability through the second pulse of precise effective polypeptide stimulation. Then, the principle of TCR-T technology is used to modify the T cells, and gene editing technology is used to knock out the immunosuppressive gene of the modified T cells. The specific killing T cells are precisely protected from inhibition in vivo, and the cytotoxicity of T cells to tumor cells is improved

The MRFFT1 cells provided by the invention can be widely used in individualized precise treatment of solid tumors.

The definitions of the professional terms are given below:
M refers to MVA virus transfection technology.
R refers to precise polypeptide second pulse technology.
FF refers to mixed polypeptide technology.
T refers to TCR-T technology.
1 refers to target knockout protection technology.

For example: MRFFT1 cells are obtained by the above M, R, FF, T, 1 technical schemes or technical means.

### The modification scheme for MRFFT1 cell:

### 1. Prediction of antigenic determinants

1) This Invention uses human peripheral blood for ctDNA sequencing or commercial engineering cell lines (for example, H1299, H226, H358, H1563, H2228, A549, Renca, mouse Lewis lung cancer (LLC) cells, CRL-6323 B16F1, CRL-2539 4T1, mouse U14 cervical cancer cells, mouse BV-2 microglioma cells, mouse G422 glioma cells, etc.) for MHC type detection and the whole exome sequencing to detect RNA mutations.
2) Antigenic determinants are predicted with by using the MHC type and gene mutation information: the mutant amino acid site is used as the target center; 8 amino acids are extended on both sides of the target center so that the polypeptides of the 17 amino acid are then used as the "potential antigenic determinants".
3) IC50 of potential antigenic determinants is analyzed by using predictive software, such that the potential antigenic determinants with IC50 < 1000 nM is considered as antigenic determinants.

### 2. Polypeptide ligation

1) IC50 at the junction of two linked antigenic determinants of any antigen is analyzed with the software mentioned above, and it is considered weak immunogenicity and can be linked when IC50 ≥ 1000 nM, while it is considered strong immunogenicity and cannot be linked when IC50 < 1000 nM.
2) Based on the above results, the weak immunogenic antigenic determinants are linked together, and IC50 at the junction is higher than that at the bilateral antigenic determinants (that is to say, the strong binding antigen should be avoided at the junction as far as possible).

### 3. Polypeptide synthesis

1) The linked polypeptides are reduced to nucleic acid sequences and codon optimization is performed.
2) Gene sequences of antigenic determinants peptides are synthesized by solid-phase synthesis or third-party technical service company.

### 4. Construction of MVA virus expressing antigenic determinants peptides.

The expression plasmid of MVA viral expressing antigenic determinants peptides is constructed from the gene sequences synthesized in the previous step and packaged with virus.

### 5. Transfection of antigen presenting cells (APC) and co-culture with PBMC

1) Antigen presenting cells (including but not limited to: peripheral blood mononuclear cells, dendritic cells, neutrophils, B lymphocytes, macrophages) are transfected by rMVA virus expressing antigenic determinants peptides.
2) APCs are collected and co-cultured at the ratio of 1:5-20 of APC: PBMC to obtain effector cells.

### 6. Screening of effective precise polypeptide and stimulating T cell with precise polypeptides

1) T cells are collected by centrifugation. Polypeptides are used as antigens to directly stimulate T cells to screen precise polypeptides.
2) To set up positive control: T cells + 100 ng/mL OKT3; and negative control: T cells + 1640 + 10% FBS + 200 U / mL IL2.
3) Evaluation criteria of precise polypeptide:
   a. If the positive and negative controls are normal, such that the data is reliable

### b. If the experimental group is significantly exceeding the negative control group, then it is an effective precise polypeptide.

4) T cells are pulsed a second time by the selected precise polypeptides.

### 7. Construction of TCR-T cells

1) The stimulated T cells are stained with CD8, CD137 and IFN-γ and sorted by flow cytometry.
2) Specific cells capable of recognizing the precise polypeptides are screened out and sequenced to determine and amplify high frequency TCR sequences.
3) Vectors for TCR gene expression are constructed and virus is packaged.
4) The original TCR gene in peripheral blood T cells is knocked out and the TCR gene obtained from the previous step is transferred into it to obtain TCR-T cells.

### 8. Knocking out the immunosuppressive signal molecules on the cell surface, and obtaining the MRFFT1 cell.

The immunosuppressive signal molecule on the cell surface includes: PD-1, Tim-3, LAG3, CTLA-4, BTLA, VISTA, CD160, 2B4 (CD244).

### 9. Construction of target cell expressing specific antigen and the survival experiment of tumor model

The beneficial effects of the Invention are as follows:
1. The MRFFT1 cells provided by the Invention use tumor antigen as mutant antigen. The MRFFT1 cells are equipped with strong target specificity which do not miss targets easily unlike the other tissues.
2. The Invention produces a high proportion of specific cells. In general, cells that recognize tumor antigens are less than 0.5% in PBMC. The percentage of specific T cell (TCR+) that can recognize tumor antigens is more than 70% after the MRFFT1 modification.
3. The MRFFT1 cells obtained in this Invention have knocked-out immunosuppressive targets such as PD1, CTLA4, TIM3 and LAG3, resulting in a higher killing frequency and unrestricted killing ability against the tumor.

### BRIEF DESCRIPTION OF DRAWINGS

FIG. 1 is a diagram of efficiency detection of APC transfected by MVA Virus; wherein, 1A refers to the control group, 1B refers to the infectious group.
FIG. 2 is a diagram of detection of MFF cell typing.
FIG. 3 is a diagram of screening of precision polypeptides.
FIG. 4 is a diagram of flow cytometry to detect the proportion of specific T cells, wherein, 4A refers to control group and 4B refers to MRFF scheme.
FIG. 5 is a diagram of TCR distribution frequency.
FIG. 6 is a diagram of the knockout of inhibitory targets, wherein, 6A refers to the situation before knockout and 6B refers to the situation after knockout.
FIG. 7 is a diagram of knockout efficiency detection of original TCR, wherein, 7A refers to the situation after knockout and 7B refers to the situation before knockout.
FIG. 8 is a diagram of expression efficiency of specific TCR, wherein, 8A refers to the transfection after 7 days; and 8B refers to the situation before transfection.
FIG. 9 is a diagram of detection of killing efficiency of the LDH release
FIG. 10 is a diagram of detection of cytokine IFN-γ release by ELISA
FIG. 11 is a diagram of survival curve of animal tumor-bearing models.

### Implementation Details:

The Invention is described below through specific embodiments. Unless otherwise specified, the technical means used in the present invention are known to those skilled in the art. In addition, the embodiments should be interpreted as illustrative rather than limiting the scope of the invention, which is limited only by the claims. For those skilled in the art, without departing from the essence and scope of the present invention, various changes or alterations in the composition and amount of materials in these embodiments are also within the scope of protection of the present invention.

The technical solution is described in detail as follows:

### 1. Prediction of antigenic determinants

1) The peripheral blood of lung cancer patients is taken for sequencing of ctDNA and HLA typing.
2) Use software to analyze the sequencing information: the mutation sites are screened by comparing the results of ctDNA sequencing and the genomes of normal cells.
3) The mutant amino acid site is used as the target center; 8 amino acids are extended on both sides of the target center so that the polypeptides of the 17 amino acid are then used as the "potential antigenic determinants".
4) IC50 of potential antigenic determinants is analyzed by using predictive software (recommended software: NetMHCpan 3.0, PickPocket, and artificial neural networks (ANN)), such that the potential antigenic determinants with IC50<1000 nM is considered as antigenic determinants.

### 2. Polypeptide ligation

1) The IC50 at the junction of any two linked antigenic determinants is analyzed by the software mentioned above, and it is consider as weak immunogenicity can be linked when IC50 is ≥ 1000 nM, while it is consider as strong immunogenicity and cannot be linked when IC50 < 1000 nM (the results of IC50 calculated by three prediction software should be consider here. When IC50 calculated by two or more software is greater than or equal to 1000 nM, it can be considered as weak immunogenicity. When IC50 calculated by two or more software is less than 1000 nM, it can be considered strong immunogenicity).
2) Based on the above results, the antigenic determinants are linked together, and IC50 at the junction is higher than that at the bilateral antigenic determinants (that is to say, the strong binding antigen should be avoided at the junction as far as possible). If necessary, use weak immunogenic peptides as ligands to separate strong immunogenic peptides; or add patients' own amino acids to the junction to reduce the possibility of producing strong antigens.

### 3. Synthesis gene sequences of encoding polypeptides

1) The linked polypeptides are reduced to nucleic acid sequences and codon optimization is performed.
   Amino acid sequence can be duplicated appropriately if the nucleic acid sequence is shorter (< 100 bps) after ligation, but attention should be paid to avoiding reverse, direct and mirror repeat sequence in gene sequence when it is reduced to gene sequence.
2) Gene sequences of antigenic determinants peptide are synthesized by solid-phase synthesis (synthesized by the third-party technical service company).

### 4. Construction of MVA virus expressing antigenic determinants peptides

1) Construction of shuttle plasmid: cloning of the synthesized antigenic determinants peptides gene sequence into pIIIdHR-P7.5 plasmid.
2) Construction of recombinant MVA virus: growing monolayer to 70%∼90% coverage, CEF cells (chicken embryo fibroblasts) or BHK-21 cells (hamster kidney fibroblasts) is infected with MVA and transfected shuttle plasmid in turn, and cultured to obtain monolayer cells. The rMVA solution is obtained by freezing, thawing and fragmentation of the monolayer cells. The rMVA solution is inoculated into RK-13 cells (rabbit kidney cells) to obtain aggregation points of RK-13 infected with rMVA, and the aggregation points are selected to screen and purify the required rMVA. After removing the wtMVA from the rMVA, rMVA is cultured in CEF or BHK-21 cell monolayer, and rMVA without K1L is screened and purified to obtain the rMVA- antigenic determinants peptide, and the rMVA-antigenic determinants peptide is amplified and isolated.

### 5. Transfection of antigen presenting cells (APC) and co-culture with PBMC

1) Antigen presenting cells (including but not limited to: peripheral blood mononuclear cells, dendritic cells, neutrophils, B lymphocytes, macrophages) are transfected by rMVA virus expressing antigenic determinants peptides.
2) Antigen presenting cells (APC) are coated into 6-24 well plates with a concentration of 1-10 x 10⁶/mL.
3) APC is infected with rMVA virus expressing antigen polypeptide with a MOI of 0.1-2. 2-24 hours after infection, 0.5-2 mL AIM V culture medium containing 100-2000 IU/mL IL-2, 10-100 ng/ml hTNF-a, 1000-5000 IU/mL IL-6 and 10-100 ng/mL IL-1β is added to each well and continue culture for 2-48 hours.
4) APCs are collected and co-cultured at the ratio of 1:5-20 of APC: PBMC, and PBMC is about 5 x 10⁷. 50 mL OKM100 medium is added to T75 cell culture flask and culture for 14 days in cell culture box at temperatures between 30 DEG C and 37 DEG C to obtain effector cells of MFF scheme.

### 6. Screening of effective precise polypeptides

Polypeptides are used as antigens to directly stimulate effector cells to screen precise polypeptides.
1) The above effector cells of MFF scheme are collected by centrifugation. T cells are collected by centrifugation at a speed of 1500 rpm for 5 minutes, then 10 mL PBS suspended cells are added and counted, and T cells are collected by centrifugation at a speed of 1500 rpm for 5 minutes, and re-suspended by 1640 + 10% FBS + 200 U / mL IL2, and the count was adjusted to 1 x 10⁶ cells / mL.
2) T cells are discharged into 96 well flat plates with a multi-channel pipette, in the amount of 200 µL per well, and the number of T cells was 2 x 10⁵. Then mutant polypeptides are added with a final concentration of 50 µg/mL, and each polypeptide is provided with three wells.
3) The inventors set up positive control: T cells + 100 ng/mL OKT3; and negative control: T cells + 1640 + 10% FBS + 200 U / mL IL2.
4) After stimulated for 24 hours at 37 DEG C with 5% CO₂, mutant polypeptides are centrifuged at a speed of 1500 rpm for 10 minutes and 140 µL supernatant is transferred to a new 96 well plate.
5) The 96-well plate is centrifuged at a speed of 1500 rpm for 10 minutes, and the samples are detected by ELISA (or stored at - 80 DEG C). ELISA system for detecting IFN -γ

1) At present, the tested ELISA kits used to detect IFN-γ include Biolegend: LEGEND MAX Human IFN-γ ELISA Kit with Pre-coated Plates (goods number: 430107) and Dako: Human IFN-γ ELISA Kit (goods number: DKW12-1000-096). Please operate strictly according to the manufacturer's instructions.
2) ELISA manual cladding system (15 plates): Human IFN- y DuoSet 15 plate (goods number: DY285B) x 1, DuoSet ELISA Ancillary Reagent Kit 2 (goods number: DY008) x 3.

Evaluation criteria of precise polypeptide:
a. if the positive and negative controls are normal, this data is reliable.
b. If the experimental group is significantly exceeding than the negative control group, the polypeptide is an effective precise polypeptide.

### 7. T cells are pulsed second time by the selected precise polypeptide

1) When PBMC is cultured in step 5 for 2-14 days, 2 x 10⁷ effector cells are taken, and precise polypeptides are added with a final concentration between 10 µg/mL and 100 µg/mL. and the cells are pulsed for 1-4h.
2) After pulsed for 4h, the cells are transferred into 6 well plates with OKM25 pre-cladding or T25 cm² culture flask, supplemented with OKM100 + 12% FBS and cultured at 37 DEG C with 5% CO₂. The cells are transferred to T75 culture flask according to the growth condition, and the cell density is kept at 1 x 10⁶ cells/mL.
3) When the T cells enter T175 flask, the culture medium is OKM200 + 5% FBS. After 10 to 14 days of culture, MRFF cells, which were obtained by the second pulse of precise polypeptide, could be obtained.

### 8. Culture and isolation of specific killer T cells of mutant antigen

1) The MRFF cells obtained in step 7 are stimulated with the selected precise polypeptide as antigen directly. After stimulated for 12-72 hours, the obtained MRFF cells are reserved.
2) The stimulated T cells are stained with CD8, CD137 and IFN-γ and sorted by flow cytometry. CD8 + CD137 + cells or CD8 + IFN-γ+ cells are selected.

### 9. Detection of TCR frequency in CD8+T cells and cloning of high frequency TCR

1) Extract the selected genomes of CD8 + CD137 + cells or CD8 + IFN-γ+ cells for TCR frequency detection to determine the sequences of high frequency TCR.
2) The mRNA of the selected cells are extracted and reverse transcribed into DNA. The gene of TCR is amplified by primers designed according to the sequence of high frequency TCR.
3) Construction of vectors for TCR gene expression and package virus.

### 10. Construction of CRISPR vector with knockout of immunosuppressive signaling molecules

1) The immunosuppressive signaling molecule on the cell surface includes: PD-1, Tim-3, LAG3, CTLA-4, BTLA, VISTA, CD160, 2B4 (CD244).
2) The inventors analyses the exons of immunosuppressive signaling molecules and find the CDS region of the gene mRNA on pubmed, and predict the knockout targets of each exon separately.
3) The forward and reverse primers needed for the synthesis of sgRNA are designed. After mixing at the ratio of 1:1, the forward and reverse primers are treated at 95 DEG C for 5 ∼ 60 min and then slowly cool down to form the DNA sequence of sgRNA .
4) The vector of CRISPR lentivirus expression is digested by double restriction enzyme digestion and linked with double-stranded DNA corresponding to sgRNA, and transferred into competent cells for cloning. After 12 hours, the monoclonal is selected for sequencing and the correct sequenced clones are retained.
5) The plasmid vector of CRISPR lentivirus carrying DNA sequence corresponding to sgRNA is extracted and packaged with virus.

### 11. Construction of CRISPR vector for TCR knockout

1) The inventors find the CDS region of the TCR gene mRNA on pubmed, analyze the conservative region of TCR, and predict the knockout targets of the conservative region.
2) The construction of TCR knockout vectors and viral packaging are completed according to steps 3 to 5 of step 10.

### 12. Construction of TCR-T that knocks out immunosuppressive signaling molecules

1) CD8 + T cells acquired in step 8 are infected with viruses acquired in steps 10 and 11. Meanwhile, the original TCR and immunosuppressive signaling molecules are knockout.
2) After knockout, CD8 + T cells are cultured in the medium for 0 - 5 days, and optimized for 3 days, after that transferred into the constructed TCR expression vector.
3) The infected CD8 + T cells are suspended with OKM100 + 12% FBS and placed on the pre-paved stimulating factor OKM-25, and record the time as day 0.
4) Observe the condition and density of the cells, and the co-cultured cells are transferred to a large culture flask and fresh medium OKM-100+12% FBS is added on the fifth day.
5) The cells are transferred from 75 cm² flask to 175 cm² flask, and the medium is OKM-200+5% FBS.
6) When cultured for 14-21 days, TCR-T or MRFFT1 cells, which knocked out immunosuppressive signal molecules, could be harvested.

### 13. Construction of target cells expressing specific antigens and survival experiment of tumor model

1) Lentiviral vectors expressing precision peptides (specific antigens) selected are constructed.
2) Lentiviral vectors expressing specific antigens are packaged into lentiviral particles to infect HLA-matched tumor cells, and stabilize over-expression of specific antigens, and the expression level and intensity are detected by flow cytometry.
3) NGS mice are inoculated with tumor cell lines stably overexpressing specific antigen peptide to establish heterotopic tumor-bearing animal models. 5 x 10⁵ tumor cells expressing specific antigens are suspended in 100 µl saline and subcutaneously injected into the right flank and rib of 30 NSG mice, respectively, and the NSG mice are numbered at the same time.
4) When the tumor grows to about 100-120 mm³, the animal models are randomly divided into three groups according to the size of tumors, and there are 5-6 mice in each group. One group is given placebo saline, one group is given 1×10⁷ T cells (control group) without genetic manipulation, and the other group was given 1×10⁷ MRFFT1 cells. The injection of cells will take place with an interval of 7 days after the first injection, and each group of mice need to be injected 3 times. The inventors record survival data continuously for 60 days and draw survival curve.

### Experimental Results:

### 1. Prediction of mutation sites and antigen epitope

Table 1 shows the predicted results of mutation sites and antigenic determinants detected by sequencing.

**Table 1 Prediction of antigenic determinants**

| peptide number | antigenic determinants | affinity with HLA (nM) |
|---|---|---|
| 1 | QELFHLFEL | 96.1 |
| 2 | KTGIGVLP | 72.3 |
| 3 | KTGIGVWA | 110.7 |
| 4 | YSWLGETLL | 82.3 |
| 5 | QYGRLYPT | 88.74 |
| 6 | AFQELFHLF | 10.9 |
| 7 | VPGNLLLS | 26.31 |
| 8 | PTGSFVLR | 121.38 |
| 9 | VYIAILSYSI | 17.78 |
| 10 | CEYNFVSPL | 36.7 |
| 11 | YSFPARVPPPL | 3.36 |
| 12 | FPARVPPPL | 32.6 |

### 2. Efficiency detection of APC transfected by MVA virus

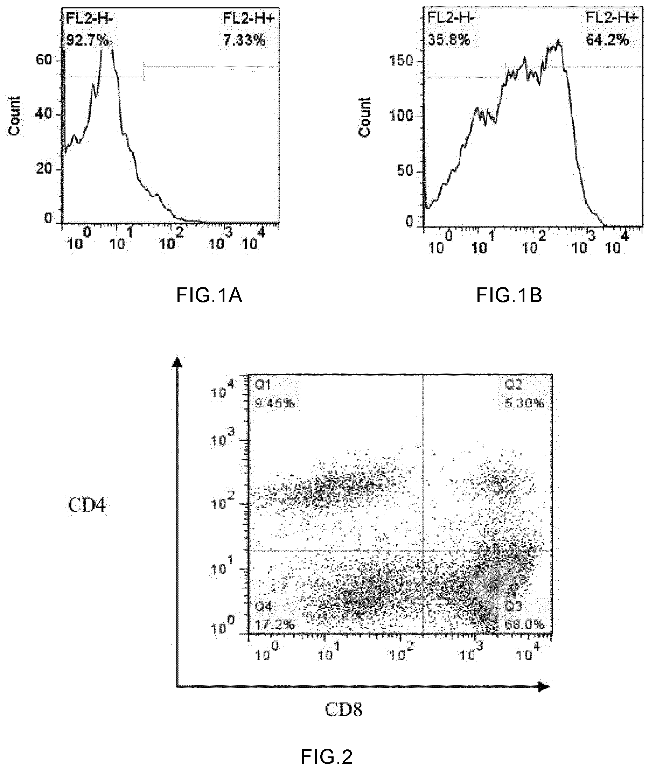

1) Antigen presenting cells (including but not limited to: peripheral blood mononuclear cells, dendritic cells, neutrophils, B lymphocytes, macrophages) are transfected by rMVA virus expressing antigenic determinants peptides.
2) Antigen presenting cells (APC) are coated into 6-24 well plates with a concentration of 1-10 x 10⁶/mL.
3) APC is infected with rMVA virus expressing antigen polypeptide with a MOI of 0.1-2. 2-24 hours after infection, 0.5-2 mL AIM V medium containing 100-2000 IU/mL IL-2, 10-100 ng/ml hTNF-a, 1000-5000 IU/mL IL-6 and 10-100 ng/mL IL-1β is added to each well and continue culture for 2-48 hours.
4) the positive rate of GFP in APC is detected by flow cytometry (as shown in Fig. 1).

### 3. Detection of MFF cell typing

After MFF cells culture, CD4 + cells and CD8 + cells are typed. As shown in Fig. 2, the percentage of CD8 + T cells is 68% and the percentage of CD4 + T cells is 9.45%.

### 4. Screening of precision peptides by MFF Cells

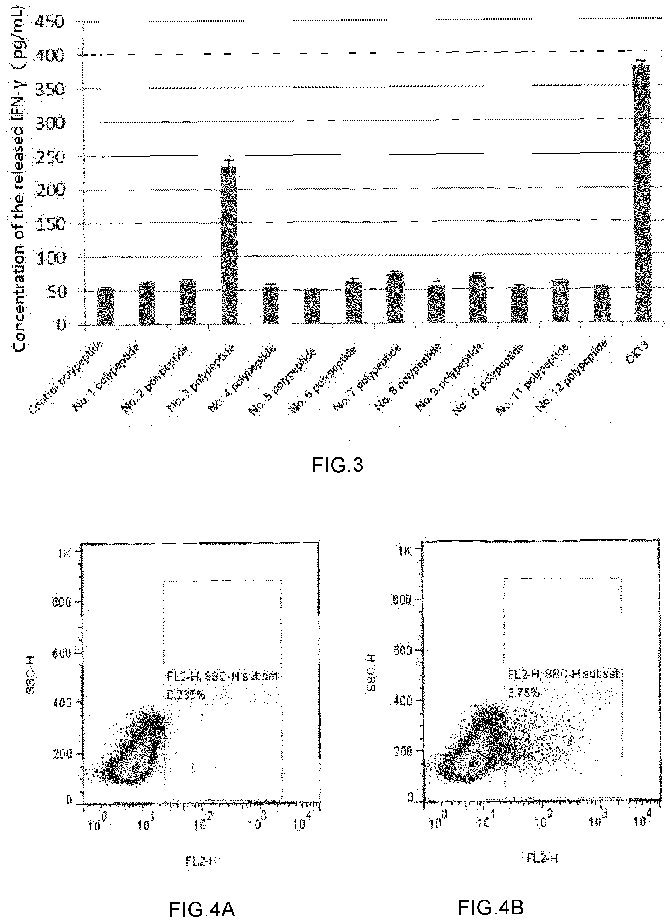

Twelve polypeptides are used to stimulate the cultured T cells respectively. Effective polypeptides are detected by detecting the secretion of IFN-γ. As shown in Fig. 3, the release of IFN-γ induced by No. 3 polypeptide is higher than that of negative control, which belonged to effective and precise polypeptide.

### 5. Identification and sorting of T cells specific to precision peptides

The MFF scheme cells are stimulated by the selected No. 3 polypeptide. The proportion of specific T cells to precise polypeptide is detected by flow cytometry. As shown in Figure 4, specific T cells are found in the black box, and the proportion of lFN-γ-releasing cells induced by polypeptide no.3 of MRFF scheme cells are significantly higher than that of non-stimulating cells (control). The results show that specific T cells for precise polypeptide can be obtained by MRFF scheme. At the same time, CD8 + IFN-γ+ cells (in the black box) are sorted by flow cytometry.

### 6. Identification and cloning of high frequency TCR

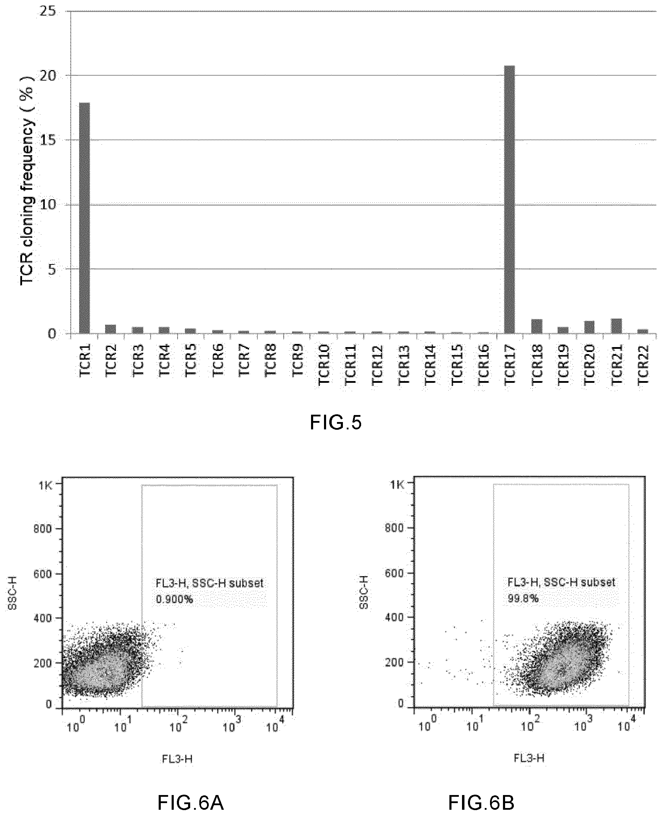

Genome extraction and sequencing of TCR are performed on the sorted cells. The distribution of TCR is shown in Fig. 5 (TOP 22 of high frequency distribution). The TCR1 and TCR17 have higher frequency distribution. This indicated that TCR is closely related to mutant antigen. According to TCR 17 sequence to amplify TCR and constructed the lentivirus expression vector.

**Table 2 Sequence situation of TCR β-chain CDR3**

| number | CDR3 DNA sequence | CDR3 amino acid sequence |
|---|---|---|
| TCR1 | TGTGCCAGCAGTGAAGGCGGGGACACCGGGGAGCTGTTTTTT | CASSEGGDTGELFF |
| TCR2 | TGTGCCAGTAGGGGGGGGGGCAACAATGAGCAGTTCTTC | CASRGGGNNEQFF |
| TCR3 | TGTGCCTGGGGATACAACACTGAAGCTTTCTTT | CAWGYNTEAFF |
| TCR4 | TGTGCCAGCAGCTTAGGGGGGGGACAGGGGCTATTGAACACTGAAGCTT TCTTT | CASSLGGGQGLLNTEAFF |
| TCR5 | TGTGCCAGCAGCGACAGCATGAACACTGAAGCTTTCTTT | CASSDSMNTEAFF |
| TCR6 | TGTGCCAGCAGTCCAGACTCCGGGACAGGGGGCGAGTACTTC | CASSPDSGTGGEYF |
| TCR7 | TGTGCCACCACGACAGAGGTGGAATATAGGTATAGCAATCAGCCCCAGC ATTTT | CATTTEVEYRYSNQPQHF |
| TCR8 | TGTGCCAGCAGCTTCCCGGATGAGGGGGACTATGGCTACACCTTC | CASSFPDEGDYGYTF |
| TCR9 | TGTGCCATCAGTGATGGTGGAAACACCATATATTTT | CAISDGGNTIYF |
| TCR10 | TGCAGTGCTAGGACAGGGGACGGGTCACCCCTCCACTTT | CSARTGDGSPLHF |
| TCR11 | TGTGCCTGGAGTGTGGCGCTGAACACTGAAGCTTTCTTT | CAWSVALNTEAFF |
| TCR12 | TGTGCCAGCAGTTTATCCGTACAGGCCTCCTCCTACGAGCAGTACTTC | CASSLSVQASSYEQYF |
| TCR13 | TGTGCCAGCAGTGACGGGACGGCCACCGGGGAGCTGTTTTTT | CASSDGTATGELFF |
| TCR14 | TGTGCCAGCAGTTACTCTCAAGGGGCCGGGGAGCTGTTTTTT | CASSYSQGAGELFF |
| TCR15 | TGTGCCAGCAGTCAGGGACTAGCAGACCAAGAGACCCAGTACTTC | CASSQGLADQETQYF |
| TCR16 | TGTGCCAGTAGGGGGGGCGCAGACAATGAGCAGTTCTTC | CASRGGADNEQFF |
| TCR17 | TGCGCCAGCAGCTCTGGGACCTATGGCTACACCTTC | CASSSGTYGYTF |
| TCR18 | TGTGCCATCGCGGTACAGGGTAATGAAAAACTGTTTTTT | CAIAVQGNEKLFF |
| TCR19 | TGTGCCAGCAGGCCCACAGGGAACACTGAAGCTTTCTTT | CASRPTGNTEAFF |
| TCR20 | TGTGCCAGCAGTTTGGGGGGGTCGACACCCCTCCACTTT | CASSLGGSTPLHF |
| TCR21 | TGTGCCGGACAGTCGGTGGTCTCGGACAGGGGTACCTACGAGCAGTACT TC | CAGQSVVSDRGTYEQYF |
| TCR22 | TGTGCCAGCAGTTTAGAACAGGGGGCGGTTCTCGACGAGCAGTACTTC | CASSLEQGAVLDEQYF |

Known TCR-α:
Amino acid sequence: 
MMKSLRVLLV ILWLQLSWVW SQQKEVEQNS GPLSVPEGAI ASLNCTYSDR
GSQSFFWYRQ YSGKSPELIM FIYSNGDKED GRFTAQLNKA SQYVSLLIRD
SQPSDSATYL CAVNFGGGKL IFGQGTELSV KPN
Base sequence:

ATGATGAAAT CCTTGAGAGT TTTACTAGTG ATCCTGTGGC TTCAGTTGAG
CTGGGTTTGG AGCCAACAGA AGGAGGTGGA GCAGAATTCT GGACCCCTCA
GTGTTCCAGA GGGAGCCATT GCCTCTCTCA ACTGCACTTA CAGTGACCGA
GGTTCCCAGT CCTTCTTCTG GTACAGACAA TATTCTGGGA AAAGCCCTGA
GTTGATAATG TTCATATACT CCAATGGTGA CAAAGAAGAT GGAAGGTTTA
CAGCACAGCT CAATAAAGCC AGCCAGTATG TTTCTCTGCT CATCAGAGAC
TCCCAGCCCA GTGATTCAGC CACCTACCTC TGTGCCGTGA ACTTCGGAGG
AGGAAAGCTT ATCTTCGGAC AGGGAACGGA GTTATCTGTG AAACCCAAT

Known TCR-β:
Amino acid sequence: 
MRIRLLCCVA FSLLWAGPVI AGITQAPTSQ ILAAGRRMTL RCTQDMRHNA

MYWYRQDLGL GLRLIHYSNT AGTTGKGEVP DGYSVSRANT DDFPLTLASA

VPSQTSVYFC ASSLSFGTEA FFGQGTRLTV V
The underlined part is the CDR3 sequence, which needs to be substituted. Substituted TCR-β:

MRIRLLCCVA FSLLWAGPVI AGITQAPTSQ ILAAGRRMTL RCTQDMRHNA

MYWYRQDLGL GLRLIHYSNT AGTTGKGEVP DGYSVSRANT DDFPLTLASA

VPSQTSVYF CASSSGTYGYTF GQGTRLTVV

The underlined part is the substituted CDR3 sequence.

### 7. Detection of the knockout efficiency of inhibitory target

The inhibitory target PD-1 of PBMC is knocked out by CRISPR technology. The sequence of sgRNA is shown in Table 3. The knockout efficiency of the inhibitory target is shown in Fig 6. The knockout efficiency of sgRNA1 is the highest which can effectively block the expression of the inhibitory signal molecule PD-1. SgRNA prefers sgRNA1 and sgRNA2. This method can also be used to knock out inhibitory signal molecules such as Tim-3, LAG3, CTLA-4, BTLA, VISTA, CD160, 2B4 (CD244), which can effectively block the expression of inhibitory signal molecules.

**Table 3 Inhibitory target sgRNA sequence**

| number | sequence 5'-3' |
|---|---|
| sgRNA1 | ACTTCCACATGAGCGTGGTCAGG |
| sgRNA2 | GCGTGACTTCCACATGAGCGTGG |

### 8. Detection of the knockout efficiency of original TCR

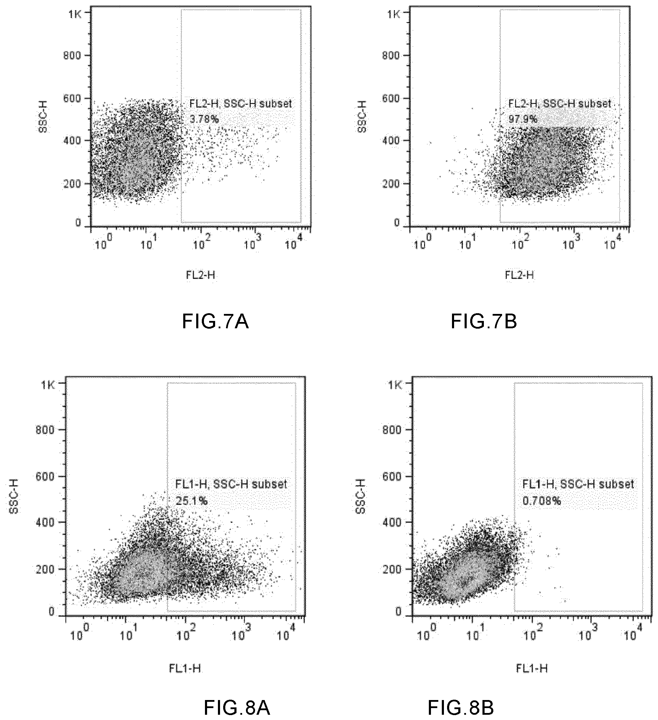

The original TCR of PBMC is knocked out by CRISPR technology. As shown in Fig 7: It can effectively reduce the expression of the original TCR. At this time, the lentivirus expressing specific TCR can be transfected.

### 9. Detection of specific TCR expression

PBMC is transfected with lentiviruses packaged with specific TCR. On the 7th day, the expression efficiency of TCR is detected by flow cytometry. As shown in Fig 8: the constructed TCR could express normally and the proportion of TCR + cells is 25.1%.

### 10. Killing effect of MRFFT1 cells on target cells.

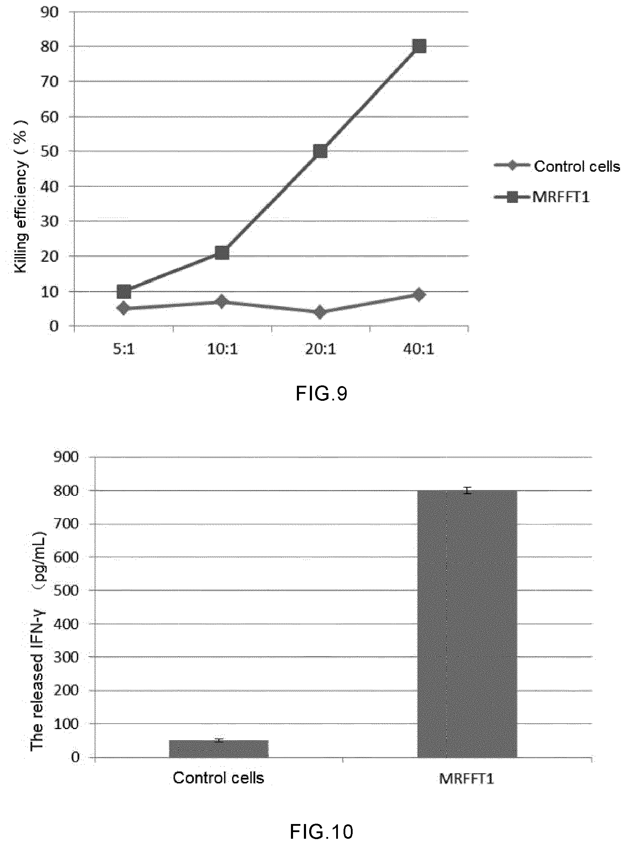

The killing efficiency of target cells derived from mutant antigen epitopes is tested by control cells and MRFFT1 cells respectively. The untreated cells are used as control cells (Mock). The results show that the killing efficiency of target cells of MRFFT1 cells is stronger than that of control cells as shown in Fig. 9.

### 11. Detection of cytokines released by MRFFT1 cells

In co-culture of tumor cells and effector cells, because effector cells can recognize mutant antigens of tumor cells, a series of cytokines will be produced. IFN-γ is one of the most important cytokines in anti-tumor effect. Fig. 10 is the detection of IFN-γ released by MRFFT1 cells when co-cultured with tumor cells. The results show that, compared with IFN-γ produced by effector cells themselves (T cells only), MRFFT1 cells could produce a large number of IFN-γ after co-culture with tumor cells. The results are consistent with the killing experiment. It is concluded that T cells expressing specific TCR, combined with knockout of inhibitory targets, can enhance the anti-tumor effect more effectively.

### 13. Construction of target cells expressing specific antigens and survival experiment of tumor model

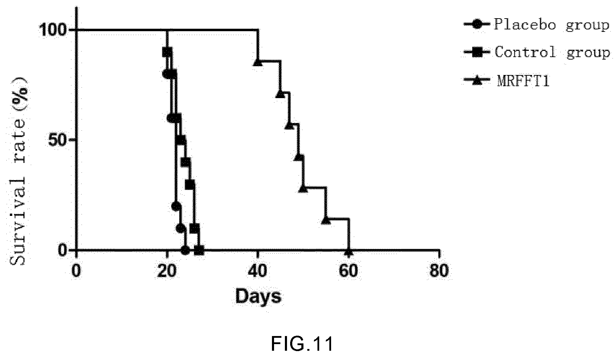

The target cell line expressing specific antigen is successfully constructed and the animal model of tumor bearing is established. The results show that (Fig. 11): MRFFT1 cells can significantly improve the survival of tumor-bearing mice.

### 14. Clinical cases

A man: 61 years old.

Disease diagnosis: poorly differentiated adenocarcinoma of both lungs; left pulmonary tuberculosis.

The first course of treatment: MRFFT1 cells are used once a month, and the number of MRFFT1 cells is 1 x 10⁹, 2 times in all;

The second course of treatment: MRFFT1 cells are used once a half month, and the number of MRFFT1 cells is 1 x 10⁹, 2 times in all;

After administration, progression-free survival is 22 months.

Other cases:

| patient number | disease diagnosis | progression-free-survival |
|---|---|---|
| 1 | Liver metastasis of gastric adenocarcinoma | From March 2016 to the present |
| 2 | gastric adenocarcinoma | From March 2016 to the present |
| 3 | lung cancer | From April 2016 to the present |
| 4 | lung adenocarcinoma | From April 2016 to the present |
| 5 | lung adenocarcinoma | From June 2016 to the present |
| 6 | esophageal cancer | From June 2016 to the present |

| | | |
|---|---|---|
| Note:" to the present" means "one day before the filling date " | | |

## Claims

1. A specie of MRFFT1 cell, prepared by the following steps:
1) screening out mutation sites by ctDNA sequencing using human peripheral blood or whole exome sequencing using tumor tissue;
2) predicting antigenic determinants based on mutation sites and synthesizing gene sequence of mutant polypeptides;
3) constructing MVA virus vector expressing mutant polypeptides and packaging the MVA virus;
4) transfecting antigen presenting cells and co-culture with PBMC to obtain MFF cells;
5) stimulating the MFF cells with the mutant polypeptides as antigens and screening out effective precise polypeptides;
6) stimulating the MFF cells with the precise polypeptides as antigens and screening out specific cells capable of recognizing the precise polypeptides, sequencing and obtaining high frequency TCR gene of specific cells;
7) knocking out an original TCR gene in peripheral blood T cells and transferring the TCR gene obtained from the previous step which could specifically bind to the precise polypeptides into it to obtain TCR-T cells; and
8) knocking out immunosuppressive signal molecules on the cell surface to obtain the MRFFT1 cell.

2. The MRFFT1 cell according to claim 1, wherein the human peripheral blood could be an engineering cell line that is commercially available.

3. The MRFFT1 cell according to claim 1, wherein the prediction of antigenic determinants uses the mutant amino acid site as the target center; 8 amino acids are extended on both sides of the target center so that the polypeptides of the 17 amino acid are then used as the "potential antigenic determinants"; and IC50 of potential antigenic determinants is analyzed by predictive software, and if IC50 < 1000nM, the potential antigenic determinants is considered as antigenic determinants.

4. The MRFFT1 cell according to claim 1, wherein the method for knocking out the original TCR gene in peripheral blood T cells and/or the immunosuppressive signal molecule on the cell surface is CRISPR technology.

5. The MRFFT1 cell according to claim 1, wherein the immunosuppressive signal molecules on the cell surface include: PD-1, Tim-3, LAG3, CTLA-4, BTLA, VISTA, CD160, 2B4 (CD244).

6. The MRFFT1 cell according to claim 1, wherein the antigen presenting cells include peripheral blood mononuclear cells, dendritic cells, neutrophils, B lymphocytes and macrophages.
